# EUROPÄISCHE PATENTSCHRIFT

(11) **EP 2 114 237 B1**
(45) Veröffentlichungstag und Bekanntmachung des Hinweises auf die Patenterteilung: **10.10.2012**
(21) Anmeldenummer: 07856842.5
(22) Anmeldetag: 18.12.2007
(51) Int. Cl.: A61B 3/10

(54) **ANORDNUNG FÜR OPHTHALMOLOGISCHE GERÄTE ZUR VERBESSERUNG VON FUNDUSBILDERN**
ARRANGEMENT FOR OPHTHALMOLOGICAL DEVICES FOR IMPROVING FUNDUS IMAGES
AGENCEMENT D'APPAREILS OPHTALMOLOGIQUES VISANT A L'AMELIORATION DES IMAGES D'OBSERVATION

(30) Priorität: 21.12.2006 DE 102006061932
(43) Veröffentlichungstag der Anmeldung: 11.11.2009
(73) Patentinhaber: Carl Zeiss Meditec AG, 07745 Jena (DE)
(72) Erfinder: KOSCHMIEDER, Ingo, 07743 Jena (DE); BIERNAT, Detlef, 07747 Jena (DE)
(74) Vertreter: Beck, Bernard
(86) Internationale Anmeldenummer: PCT/EP2007/011115
(87) Internationale Veröffentlichungsnummer: WO 2008/077532

(56) Entgegenhaltungen:
- WO-A-03/000153
- WO-A-03/105678
- DE-A1- 19 638 263
- DE-A1-102004 037 558
- JP-A- 2003 116 791
- US-A- 4 730 910
- US-A1- 2006 203 196

## Beschreibung

Die Erfindung bezieht sich auf eine Anordnung für ophthalmologische Geräte zur Verbesserung von Fundusbildern, insbesondere hinsichtlich der Freiheit von störenden Reflexen und der Gewährleistung einer gleichmäßigen und schattenfreien Ausleuchtung der Bilder sowie einer farbgetreuen Darstellung derselben.

Zur Darstellung der Retina sind unterschiedliche ophthalmologische Diagnosegeräte vorgesehen, so z.B. Ophthalmoskope, Scanner oder auch Funduskameras. Bei den Funduskameras wird die Retina über eine meist asphärische Frontoptik in eine Zwischenbildebene abgebildet und durch ein Hauptobjektiv und eine nachfolgende Optik in verschiedenen Winkelauflösungen über Okulare betrachtet bzw. mittels Foto- oder CCD-Kameras aufgenommen.

Dabei erfolgt die Beleuchtung des Augenhintergrundes über eine im Gerät befindliche Beleuchtungseinheit, welche eine ringförmige Pupille am Rand der Augenpupille erzeugt und dabei die Retina gleichmäßig ausleuchtet. Durch die geometrische Pupillentrennung von Beleuchtung und Beobachtung in der Augenpupille gelingt es, an der Hornhaut die Strahlengänge weitgehend zu entkoppeln und reflex- bzw. streulichtfreie Abbildungen zu erzielen.

Bei dieser Anordnung ergibt sich jedoch infolge der koaxialen Strahlenführung am Ophthalmoskopobjektiv zwangsläufig ein kräftiger zentraler Reflex in der Bildmitte. Maßnahmen zur Unterdrückung dieses Reflexes sind konstruktiv aufwändig und kostenintensiv. Das Ergebnis dieser Maßnahmen stellt deshalb ein wichtiges Qualitätsmerkmal einer heutigen Funduskamera dar.

Eine übliche Methode zur Reflexunterdrückung beinhaltet eine gezielte Abblendung des zentralen Bereiches einer optischen Fläche, welche auf der optischen Achse des optischen Systems liegt, z.B. durch einen schwarzen Lackpunkt. Nachteilig hierbei ist jedoch die Notwendigkeit einer genauen Justierung dieses als Blende wirkenden Punktes nach Größe und Position. Ein weiterer Nachteil besteht in der Schattenwirkung dieses Punktes, welche dann unter Umständen auf der Retina deutlich sichtbar wird. Ursache dafür ist die endliche Ausdehnung der Lichtquelle (Größe der Lampenwendel, Plasmawolke der Blitzröhre) gegenüber dem Schwarzpunkt und dessen Nähe zur Leuchtfeldblende.

Eine solche Lösung ist aus der DE 196 38 263 bekannt.

Eine weitere Möglichkeit, bereits die Entstehung des Reflexes zu verhindern, besteht darin, eine Frontoptik mit schräg verkippten optischen Flächen nach dem Quantar-Prinzip (US-PS 4,730,910) vorzusehen. In diesem Falle wird der zentrale Reflex soweit seitlich ausgelenkt, dass er nicht mehr in die Beobachtungsapertur gelangen kann. Das optische Design einer solchen Anordnung ist jedoch sehr aufwändig, weil durch das Verkippen der Flächen der Frontoptik einerseits der Reflex abgelenkt wird, andererseits entstehen aber auch zusätzliche Bildfehler, welche unbedingt kompensiert werden müssen, damit die optische Leistung des Systems erhalten bleibt.

Eine Kompensation erfordert zusätzliche optische Bauelemente, wie Keile oder torische Linsen und/oder sehr spezielle Kompensationsrechnungen mit den übrigen optischen Bauteilen. Auf diese Weise wird das optische Design sehr komplex und aufwändig. Eine solche Anordnung ist in der DE-PS 1 0316416 beschrieben. Hierin ist auch ein optisches System beschrieben, bei dem auf die Ausblendung bestimmter Lichtanteile innerhalb der Beleuchtungsoptik verzichtet wird. An Stelle einer Ophthalmoskoplinse wird ein mehrlinsiges Objektiv verwendet, dessen Linsen so gegeneinander verkippt sind, dass die direkten Reflexe an den optischen Grenzflächen nicht in die Apertur der Beobachtung gelangen. Bei diesem System werden Farblängsfehler und Farbquerfehler erzeugt, die aufwändig kompensiert werden müssen. Bei Anwendungen mit sehr kleinen Strahldurchmessern, wie z.B. bei Laseranwendungen, wirken sich auch die große Anzahl von optischen Grenzflächen und die langen Glaswege im Objektiv nachteilig aus. Durch Verunreinigungen an den Grenzflächen kann sich die Intensität stark verringern und störendes Streulicht entstehen.

Es ist ferner bekannt, in optischen Systemen, z.B. in Teleskopen, eine so genannte adaptive Optik vorzusehen, um die optische Leistungsfähigkeit und Bildqualität gerade unter den Bedingungen vorliegender Störungen oder Bildfehler bis hin zu Aberrationen höherer Ordnung zu verbessern. Sind beispielsweise die Bildfehler und Störungen bekannt, so kann mit Hilfe eines verstellbaren optischen Bauteils, beispielsweise eines aus vielen kleinen, separat verstellbaren Elementen bestehenden Spiegels, die Wellenfront gezielt korrigiert werden. Es können auf diese Weise fast ideale Abbildungsverhältnisse realisiert werden.

Die Anwendung der adaptiven Optik besitzt u.a. folgende Vorteile: Eine Kompensation auch zeitlich variabler Fehler und auch beliebiger oder sehr individueller und komplexer Bildfehler kann vorgenommen werden.

Der Einsatz adaptiver Optik für die Erhöhung der Auflösung von Bildern der Retina wurde bereits in EP-PS 1 401 326, US-PS 6,582,079 und JP-PS 2003-116792 beschrieben. Diese Anwendungen sind vor allem darauf gerichtet, in einer Funduskamera die erreichbare Grenzauflösung am Augenhintergrund zu erhöhen, indem Bildfehler des klassischen Gerätesystems und gegebenenfalls, nach erfolgter Bestimmung, auch die Bildfehler des Patientenauges kompensiert werden. Damit lassen sich sonst nicht sichtbare Details, wie Zäpfchen und Stäbchen, in der Retina sichtbar machen. Nachteilig sind dabei jedoch das sehr eingeschränkte Sichtfeld sowie die Notwendigkeit, einen Eyetracker zum Ausgleich der immer vorhandenen Augenbewegungen des Patienten verwenden zu müssen.

Bekannt ist weiterhin aus "Laser Focus World", August 1998, 18 bis 22, mit Hilfe einer adaptiven Optik die Aberrationen eines menschlichen Auges zu kompensieren, um hochauflösende Bilder der Retina für medizinische Untersuchungen zu erhalten.

So liegt der Erfindung die Aufgabe zu Grunde, eine Anordnung zur Verbesserung von Fundusbildern zu schaffen, mit welcher bei Gewährleistung einer schattenfreien und gleichmäßigen Ausleuchtung und bei Vermeidung störender Reflexe eine Vereinfachung der Kompensation der Kippfehler des Abbildungssystems und ein kostengünstiger Aufbau einer Funduskamera unter Beibehaltung einer hohen Leistungsfähigkeit erzielt werden.

Erfindungsgemäß wird diese Aufgabe bei einer nach dem Oberbegriff des ersten Anspruches gestalteten Anordnung mit den im kennzeichnenden Teil offenbarten Mitteln gelöst. In den Unteransprüchen sind Einzelheiten und weitere Ausgestaltungen der Erfindung sowie Anwendungen beschrieben.

Dabei ist es vorteilhaft, dass zusätzlich Mittel zur Kompensation der Bildfehler des Patientenauges vorgesehen sind.

Vorteilhaft kann auch mindestens ein Element des optischen Systems als Freiformfläche ausgebildet sein.

Es ist von Vorteil, wenn als Mittel zur Erzeugung von Steuersignalen für die Steuerung der adaptiven Elemente des Spiegels mindestens ein Wellenfrontsensor zur Wellenfrontbestimmung innerhalb des optischen Designs vorgesehen ist.

Es ist ferner vorteilhaft, wenn mindestens ein Wellenfrontsensor zur Echtzeitbestimmung der Wellenfront vorgesehen ist. Eine vorteilhafte Ausführung der Erfindung ergibt sich, wenn Mittel zur Bildverarbeitung per Software an einem Bildsensor vorgesehen sind, mit welchen Steuersignale für die Steuerung der adaptiven Elemente mindestens eines Spiegels gewonnen werden.

Als Beleuchtungsquelle sind vorteilhaft eine oder mehrere LED vorgesehen, die im kontinuierlichen und/oder im gepulsten Betrieb arbeiten.

Vorteilhaft ist es weiterhin, dass Mittel zur Erzeugung von sequentiellen Lichtpulsen in verschiedenen Spektralbereichen und zur synchronisierten Bildgewinnung mittels eines Bildsensors und entsprechender Bildaufbereitung zur Darstellung und Speicherung eines Colorbildes oder beliebiger anderer Kombinationen sowie monochromer Einzelbilder vorgesehen sind.

Ferner ist es von Vorteil, dass Mittel vorhanden sind zur selektiven Kompensation von chromatischen Aberrationen durch mindestens ein verstellbares optisches Element, welches synchron mit den Lichtpulsen seine Position ändert (z.B. Shiftlinse).

Vorteilhaft können auch Mittel zur selektiven Kompensation von chromatischen Aberrationen durch synchron mit den Lichtpulsen eingestellte Elemente mindestens eines adaptiven optischen Bauelementes vorgesehen werden.

Von Vorteil ist es auch, wenn mindestens ein optisches Element als spektraler Farbteiler ausgestaltet ist.

Eine räumlich günstige Anordnung ergibt sich, wenn der Abbildungsstrahlengang und/oder Beleuchtungsstrahlengang gewinkelt ist.

Die Erfindung ist mit Vorteil anwendbar in Geräten zur Laserkoagulation der Netzhaut bei unterschiedlichen Wellenlängen, insbesondere zur Multi-Color-Laserkoagulation, sowie in Geräten zur Durchführung der optischen Kohärenz Tomographie (OCT).

Vorteilhaft ist auch eine die Anwendung in Geräten für die Durchführung von Diagnose und Therapie am Auge mittels Ultra-Kurzpuls-Lasern, insbesondere mit Impulsen im Femtosekundenbereich, sowie in Geräten zum Hyper-Spektral-Imaging bzw. bei der multispektralen Abbildung zur Diagnose von Funktionen der Retina.

Eine Anwendung in Geräten zur Fundusreflektometrie und bei Punkt- oder Linien-Scannern zur Diagnose und/oder Therapie an der Retina ist vorteilhaft.

Weiterhin ist eine Anwendung in Geräten von Vorteil, welche auf konfokaler Basis arbeiten, insbesondere bei konfokalen Laserscannern.

Bei der Dokumentation von Befunden am Auge sollen scharfe, detailreiche Bilder mit hohem Kontrast erzielt werden. Dem entgegen stehen Unzulänglichkeiten und Abbildungsfehler des optischen Beobachtungs-Systems bzw. des optischen Systems des Auges im Falle von Bildaufnahme aus dem Augeninneren (Augenlinse, Glaskörper, Augenhintergrund) sowie die willkürlichen und unwillkürlichen Augenbewegungen, welche letztlich in Bildunschärfe und Kontrastverschlechterung münden. Unzulänglichkeiten, z.B. Abbildungsfehler, des optischen Beobachtungssystems bzw. des optischen Systems des Auges lassen sich durch eine geeignete Ausführung und Auslegung des Beobachtungssystems durch den hier vorgeschlagenen Weg kompensieren. Die Augenbewegungen führen zu einer Bewegungsunschärfe, die bei zu langen Belichtungszeiten während der Bildaufnahme alle optischen Kompensationsaufnahmen zunichte macht. Zur Unterdrückung dieser Bewegungsunschärfe muss mittels einer hinreichend energiereichen Lichtquelle kurz (im Bereich weniger Millisekunden) belichtet werden, beispielsweise durch eine geeignete Blitzlampe. Diese Lösung wird in der Mehrzahl der bekannten Funduskameras eingesetzt. Für Farbaufnahmen wird das tageslichtähnliche Spektrum des Blitzlichtes in Kombination mit einem farbtauglichen Bildsensor, z.B. mit einem Flächensensor mit aufgebrachten Farbfiltern, genutzt. Nachteil dieser Lösung ist, dass die Spektralcharakteristik dieser fest aufgebrachten Farbfilter nicht hinreichend genau offen gelegt ist und vor allem nachträglich nicht verändert werden kann. Somit sind eine optimale Belichtungssteuerung für die einzelnen Farben und ein an das Objekt angepasstes Farbmanagement nur eingeschränkt möglich.

Bei monochromen Techniken wird der gewünschte Spektralanteil aus dem weißen Blitzlicht mittels Farbfilter herausgefiltert. Diese Lösung ist energetisch nicht effizient. Eine bessere Lösung stellt hier der Einsatz von LED sowohl im kontinuierlichen als auch im Pulsbetrieb dar. Es werden LED's verschiedener Emissionswellenlängen, z.B. Rot, Grün und Blau für Farbaufnahmen, benutzt. Bei monochromen Techniken werden eine oder mehrere LED mit geeigneten Wellenlängen verwendet. Durch das quasi verzögerungslose Ein- und Ausschalten der LED können hinreichend kurze Belichtungszeiten erreicht werden. Die spektralen Charakteristika der LED sind ausreichend genau bekannt und können energetisch effizient durch Filter beeinflusst werden, womit ein optimales Farbmanagement einfacher realisierbar ist. Bei der Aufnahme von Färbbildern erfolgt der Bildeinzug für Rot, Grün und Blau in sinnvoller Weise sequentiell, d.h. als Bildaufnehmer wird ein S/W-Bildaufnehmer (Schwarz/Weiß-Bildaufnehmer) hoher Empfindlichkeit eingesetzt, und mittels gepulster LED wird nacheinander das jeweilige Farbteilbild eingezogen. Durch die Wahl der Impulslängen bzw. Impulshöhen kann eine optimale Aussteuerung für die Farbteilbilder erreicht werden. Bei kontinuierlicher Beleuchtung zum Zwecke der Einstellung auf das Untersuchungsobjekt kann durch geeignete Wahl der Intensitäten der eingesetzten LED eine sinnvolle, dem Objekt angepasste spektrale Lichtmischung erreicht werden.

Durch den sequentiellen Betrieb können weiterhin chromatische Aberrationen synchron zu den jeweiligen spektralen Lichtpulsen selektiv beeinflusst werden. Beispielsweise kann mit spezifischen Einstellungen eines verwendeten adaptiven Elementes während eines zugeordneten farbigen Lichtpulses eine optimale Korrektur erzielt werden. Eine ähnliche Wirkung lässt sich mit einem optischen Element erzielen, das gezielt in seiner Position im Strahlengang verändert wird, z.B. in Form einer Shiftlinse. Auf diese Weise lässt sich das optische Design hinsichtlich der chromatischen Korrekturen vereinfachen, da nicht alle Korrekturmaßnahmen gleichzeitig zur Anwendung kommen müssen.

Bei der Multi-Color-Laserkoagulation ist die Farbfehler- und Reflexfreiheit besonders wichtig, da die unterschiedlichen Wellenlängen keine Ablagen in der Anwendungsebene erzeugen dürfen. Durch die Beseitigung der Abbildungsfehler wird die registrierte, ortsgenaue Koagulation ermöglicht. Durch die reduzierte Zahl optischer Flächen ergeben sich weitere Transmissionsvorteile.

Bei der optischen Kohärenztomographie kommt die Farbfehler - und Reflexfreiheit bei der Kombination mit einer Funduskamera ebenfalls positiv zur Wirkung, da eine zusätzliche Korrektur für das erweiterte Einsatzspektrum der Lichtquellen (IR und VIS) entfällt. Ungewollte Interferenzen durch Mehrfachreflexionen und Streulicht werden vermieden.

Bei der Kombination mit Lasersystemen im ultrakurzen Zeitbereich (Femtosekunden- Laser) ist die Farbfehlerfreiheit besonders vorteilhaft, da zusätzliche Korrekturen zur Verkürzung der Pulsbreiten entfallen können.

Beim Hyper-Spektral-Imaging bzw. bei der spektralen Abbildung der Retina ist die Farbfehlerfreiheit des optischen Systems von Vorteil, da zusätzliche Elemente zur Korrektur der erweiterten spektralen Bandbreite entfallen können.

Bei der Fundusreflektometrie wird durch die Vermeidung von Störlicht ein artefaktfreies Bild erzielt.

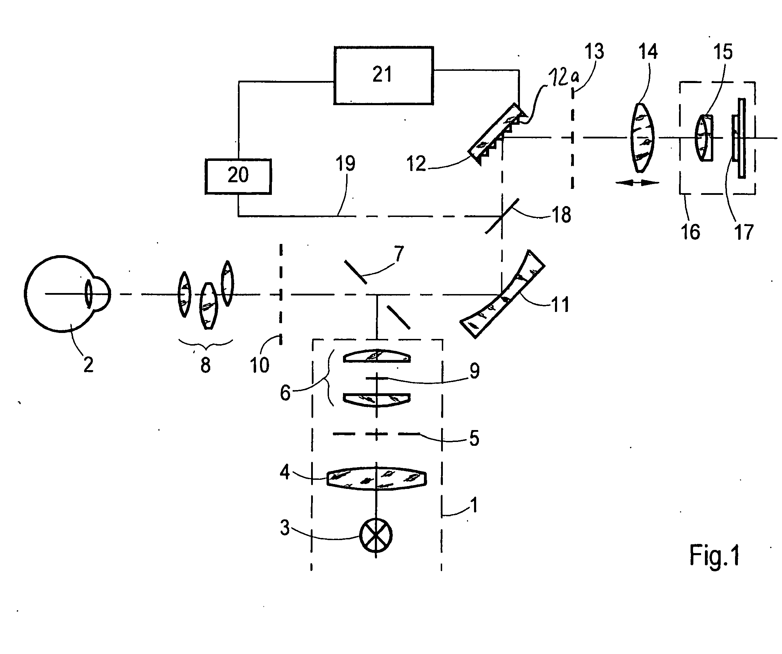

Die Erfindung soll nachstehend an einem Ausführungsbeispiel näher erläutert werden. In der Zeichnung ist das Blockschaltbild eines Ausführungsbeispiels der erfindungsgemäßen Anordnung dargestellt.

Die erfindungsgemäße optische Anordnung für ophthalmologische Geräte zur Verbesserung von Fundusbildern, insbesondere von Bildern der Retina, umfasst in einem Gehäuse eine Beleuchtungseinheit 1 und ein optisches Abbildungssystem zur weitestgehend fehlerfreien Abbildung der Retina auf einem Bildsensor einer CCD-Kamera.

Die Beleuchtung des zu untersuchenden Auges 2 des Patienten erfolgt bei der Anordnung mit Hilfe der Beleuchtungseinheit 1 ausgehend von einer geeigneten Lichtquelle 3 über einen Kondensor 4, welcher eine Ringblende 5 gleichmäßig ausleuchtet. Mit Hilfe einer Beleuchtungsoptik 6 wird die Ringblende 5 über einen mit einer zentralen Öffnung oder Bohrung versehenen kreisringförmigen Spiegel 7 und ein Ophthalmoskopobjektiv 8, welches hier verkippte und/oder dezentrierte Linsen umfasst, in die Pupille des Auges 2 als ringförmiges Leuchtfeld abgebildet, durch welches die Retina des Auges 2 gleichmäßig ausgeleuchtet wird. Die Beleuchtungsoptik 6 umfasst einen so genannten Schwarzpunkt 9, welcher für den Fall des Einsatzes einer nicht verkippten und dezentrierten Optik eine Ausblendung eines Teils des Beleuchtungslichtes bewirkt, welches an der nicht verkippten oder dezentrierten Ophthalmoskoplinse reflektiert werden würde, und bewirkt damit die weitgehende Unterdrückung störender Reflexe am Ophthalmoskopobjektiv 8 realisiert. Die Ausblendung des zentralen Teiles des Beleuchtungsstrahlenganges erfolgt durch die geometrische Auslegung der Ringblende und des Lochspiegels (Loch reflektiert kein Beobachtungslicht). Durch diese Beobachtungspupille verläuft der Beobachtungs- oder Abbildungsstrahlengang. Ein Schwarzpunkt ist beim Einsatz verkippter und dezentrierter Frontlinsen nicht notwendig und kann zur Vereinfachung des Designs entfallen.

Die beleuchtete Retina wird durch das aus einer oder mehreren verkippt und/oder dezentriert angeordneten Linsen bestehende Ophthalmoskopobjektiv 8 in einer ersten Bildebene 10 vor dem Spiegel 7 abgebildet. Dieses Zwischenbild der Retina wird dann in einem vorteilhaft mehrfach geknickten Strahlengang über einen abbildenden Spiegel 11 und einen adaptive Elemente 12a umfassenden, adaptiven Spiegel 12 in eine zweite Zwischenbildebene 13 abgebildet. Unterschiedliche Positionen, die auf Grund von Fehlsichtigkeit des zu untersuchenden Auges bedingt sind, können mit einer im Abbildungsstrahlengang der zweiten Zwischenbildebene 13 nachgeordneten Fokussieroptik 14 vorkompensiert bzw. ausgeglichen werden. Durch ein Kameraobjektiv 15 einer geeigneten Kamera 16, vorzugsweise einer CCD-Kamera, wird die Retina auf einem Sensor 17 in der Kamerabildebene abgebildet.

Wie aus der Abbildung ersichtlich, ist im Abbildungsstrahlengang zwischen dem abbildenden Spiegel 11 und dem adaptiven Spiegel 12 ein weiterer Spiegel 18 angeordnet, welcher eine Ausblendung eines Messstrahlenganges 19 auf einen Wellenfrontsensor 20 realisiert, um damit die Durchführung einer Wellenflächenprüfung zu ermöglichen, bei welcher die Abweichungen der deformierten Wellenfläche im Abbildungsstrahlengang der Anordnung von einer idealen oder gewünschten Wellenfläche bestimmt werden.

Der Wellenfrontsensor 20 ist mit einem Rechner 21 verbunden, welcher Steuersignale für die entsprechende Einstellung der adaptiven Elemente 12a des adaptiven Spiegels 12 zur Kompensation der durch das Auge und durch den Abbildungsstrahlengang bedingten Aberrationen, so dass ein weitestgehend von Abbildungsfehlern freies Bild der Retina auf dem Sensor 17 der Kamera 16 entsteht, welches dann durch den Arzt ausgewertet und weiterverarbeitet werden kann.

Durch einen solchen strukturierten bzw. adaptiven Spiegel 12 können z.B. im Beleuchtungsstrahlengang Unsymmetrien vorgehalten werden, so dass die Anforderungen an das Abbildungssystem zur reflexfreien Ophthalmoskopie geringer werden und ein einfachere Herstellung des betreffenden optischen Designs erreicht wird. Durch die Verwendung derartiger Spiegel ist auch eine Anpassung von Feldgröße und Pupille an unterschiedliche Applikationsaufgaben möglich.

Adaptive Spiegel können insbesondere auch dazu verwendet werden, gerätespezifische Fehler oder auch Augenfehlsichtigkeiten höherer Ordnung auszugleichen, womit ein optimal korrigiertes Fundusbild ermöglicht wird und z.B. dem Arzt Vorteile bei einer Diagnose verschafft werden. Vorteilhaft ist in einem solchen Falle, dass das adaptive Element in der Nähe der Gerätepupille angeordnet.

Vorgestellt wird eine Anordnung zur Verbesserung von optischen Bildern der Retina, die mit Hilfe von adaptiven Bauelementen die Bildfehler im optischen Design des Gerätes kompensiert. Diese Methode ist besonders wirksam in Kombination mit speziellen Designvarianten auf der Basis dezentrierter und verkippter optisch wirksamer Flächen.

### Bezugszeichenliste

- 1: Beleuchtungseinheit
- 2: Auge
- 3: Lichtquelle
- 4: Kondensor
- 5: Ringblende
- 6: Beleuchtungsoptik
- 7: kreisringförmiger Spiegel
- 8: Ophthalmoskoopobjektiv
- 9: Schwarzpunkt
- 10: erste Bildebene
- 11: abbildender Spiegel
- 12: adaptiver Spiegel
- 12a: adaptive Elemente
- 13: Zwischenbildebene
- 14: Fokussieroptik
- 15: Kameraobjektiv
- 16: Kamera
- 17: Sensor
- 18: Spiegel
- 19: Messstrahlengang
- 20: Wellenfrontsensor
- 21: Rechner

## Patentansprüche

1. Optische Anordnung für ophthalmologische Geräte zur Verbesserung von Fundusbildern, insbesondere zur Abbildung der Retina, umfassend zur Unterdrückung störender Reflexe
- in einem Gehäuse ein optisches Beleuchtungs- und Abbildungssystem mit verkippten und/oder dezentrierten, brechenden und/oder reflektierenden Flächen oder Anordnungen von nicht verkippten bzw. dezentrierten optischen Flächen mit Schwarzpunkt-Design im Beleuchtungssystem,
wobei
- in einem Abbildungsstrahlengang eine mindestens einen abbildenden Spiegel (11; 12) umfassende Abbildungsoptik vorgesehen ist, wobei zur Kompensation von Bildfehlern der Abbildungsoptik mindestens ein reflektierendes, brechendes, elektronisch strukturierbares oder adaptives optisches Element, z.B. ein Spiegel (12) mit verstellbaren, adaptiven Elementen (12a), vorgesehen ist.
- und Mittel zur Erzeugung von Steuersignalen für die Steuerung der adaptiven Elemente (12a) des mindestens einen optischen Elementes (12) vorgesehen sind
**dadurch gekennzeichnet,**
- **dass** als Beleuchtungsquelle eine oder mehrere LED vorgesehen sind, die im kontinuierlichen und/oder im gepulsten Betrieb arbeiten,
- und **dass** Mittel zur Erzeugung von sequentiellen Lichtpulsen in verschiedenen Spektralbereichen und zur synchronisierten Bildgewinnung mittels eines Bildsensors und entsprechender Bildaufbereitung zur Darstellung und Speicherung eines Colorbildes oder beliebiger anderer Kombinationen sowie monochromer Einzelbilder vorgesehen sind.

2. Anordnung nach Anspruch 1, **dadurch gekennzeichnet, dass** zusätzlich Mittel zur Kompensation der Bildfehler des Patientenauges (2) vorgesehen sind.

3. Anordnung nach Anspruch 1 oder 2, **dadurch gekennzeichnet, dass** mindestens ein Element des optischen Systems als Freiformfläche ausgebildet ist.

4. Anordnung nach einem der Ansprüche 1 bis 3, **dadurch gekennzeichnet, dass** als Mittel zur Erzeugung von Steuersignalen für die Steuerung der adaptiven Elemente (12a) des Spiegels (12) mindestens ein Wellenfrontsensor zur Wellenfrontbestimmung innerhalb des optischen Design vorgesehen ist.

5. Anordnung nach Anspruch 4, **dadurch gekennzeichnet, dass** mindestens ein Wellenfrontsensor zur Echtzeitbestimmung der Wellenfront vorgesehen ist.

6. Anordnung nach Anspruch 4, **dadurch gekennzeichnet, dass** Mittel zur Bildverarbeitung per Software an einem Bildsensor vorgesehen sind, mit welchen Steuersignale für die Steuerung der adaptiven Elemente (12a) mindestens eines Spiegels (12) gewonnen werden.

7. Anordnung nach Anspruch 1, **dadurch gekennzeichnet, dass** Mittel vorhanden sind zur selektiven Kompensation von chromatischen Aberrationen durch mindestens ein verstellbares optisches Element, welches synchron mit den Lichtpulsen seine Position ändert (z.B. Shiftlinse).

8. Anordnung nach einem der Ansprüche 1 bis 7, **dadurch gekennzeichnet, dass** Mittel zur selektiven Kompensation von chromatischen Aberrationen durch synchron mit den Lichtpulsen eingestellte Elemente mindestens eines adaptiven optischen Bauelementes vorgesehen sind.

9. Anordnung nach einem der Ansprüche 1 bis 8, **dadurch gekennzeichnet, dass** mindestens ein optisches Element als spektraler Farbteiler ausgestaltet ist.

10. Anordnung nach einem der vorangehenden Ansprüche, **dadurch gekennzeichnet** das der Abbildungsstrahlengang und/oder Beleuchtungsstrahlengang gewinkelt ist.

11. Anwendung einer Anordnung nach einem der Ansprüche 1 bis 10 in Geräten zur Laserkoagulation der Netzhaut bei unterschiedlichen Wellenlängen, insbesondere zur Multi-Color-Laserkoagulation.

12. Anwendung einer Anordnung nach einem der Ansprüche 1 bis 10 in Geräten zur Durchführung der optischen Kohärenz Tomographie (OCT),
und/oder in Geräten für die Durchführung von Diagnose und Therapie am Auge mittels Ultra-Kurzpuls-Lasern, insbesondere mit Impulsen im Femtosekundenbereich,
und/oder in Geräten zum Hyper-Spektral-Imaging bzw. bei der multispektralen Abbildung zur Diagnose von Funktionen der Retina,
und/oder in Geräten zur Fundusreflektometrie,
und/oder bei Punkt- oder Linien-Scannern zur Diagnose und/oder Therapie an der Retina,
und/oder in Geräten, die auf konfokaler Basis arbeiten, insbesondere bei konfokalen Laserscannern.

## Claims

1. Optical arrangement for ophthalmological devices for improving fundus images, more particularly for imaging the retina, comprising the following for suppressing interfering reflections:
- an optical illumination and imaging system in a housing, with tilted and/or decentred, refracting and/or reflecting surfaces or arrangements of not tilted and/or decentred optical surfaces with black-point design in the illumination system, wherein
- provision is made in an imaging beam path for an imaging optical system comprising at least one imaging mirror (11; 12), wherein, in order to compensate for image defects of the imaging optical system, provision is made for at least one reflecting, refracting, electronically structurable or adaptive optical element, e.g. a mirror (12) with adjustable, adaptive elements (12a),
- and provision is made for means for generating control signals for controlling the adaptive elements (12a) of the at least one optical element (12),
**characterized**
- **in that** one or more LEDs, which operate with continuous and/or pulsed operation, are provided as illumination source,
- and **in that** provision is made for means for generating sequential light pulses in various spectral ranges and for synchronized image acquisition by means of an image sensor and for appropriate image processing for displaying and storing a colour image or any other combination as well as monochrome individual images.

2. Arrangement according to Claim 1, **characterized in that** provision is additionally made for means for compensating for the aberrations of the patient eye (2).

3. Arrangement according to Claim 1 or 2, **characterized in that** at least one element of the optical system is embodied as a freeform surface.

4. Arrangement according to one of Claims 1 to 3, **characterized in that** at least one wavefront sensor for determining the wavefront within the optical design is provided as means for generating control signals for controlling the adaptive elements (12a) of the mirror (12).

5. Arrangement according to Claim 4, **characterized in that** provision is made for at least one wavefront sensor for determining the wavefront in real time.

6. Arrangement according to Claim 4, **characterized in that** provision is made for means for image processing by software at an image sensor, by means of which means control signals are obtained for controlling the adaptive elements (12a) of at least one mirror (12).

7. Arrangement according to Claim 1, **characterized in that** means are present for selective compensation of chromatic aberrations by at least one adjustable optical element (e.g. shift lens), which changes its position synchronously with the light pulses.

8. Arrangement according to one of Claims 1 to 7, **characterized in that** means are provided for selective compensation of chromatic aberrations by means of elements of at least one adaptive optical component, set in synchronized fashion with the light pulses.

9. Arrangement according to one of Claims 1 to 8, **characterized in that** at least one optical element is embodied as spectral colour splitter.

10. Arrangement according to one of the preceding claims, **characterized in that** the imaging beam path and/or the illumination beam path is angled.

11. Use of an arrangement according to one of Claims 1 to 10 in devices for laser photocoagulating the retina at different wavelengths, more particularly for multi-colour laser photocoagulation.

12. Use of an arrangement according to one of Claims 1 to 10 in devices for carrying out optical coherence tomography (OCT),
and/or in devices for carrying out diagnosis and therapy on the eye by means of ultra-short-pulse lasers,
more particularly with pulses in the femtosecond range,
and/or in devices for hyper-spectral imaging or in multispectral imaging for diagnosis of functions of the retina,
and/or in devices for fundus reflectometry,
and/or in point or line scanners for diagnosis and/or therapy on the retina,
and/or in devices that operate on a confocal basis, more particularly in confocal laser scanners.

## Revendications

1. Dispositif optique pour appareils ophtalmologiques destinés à améliorer les images de fond de l'oeil, notamment pour la formation d'image de la rétine, comprenant, pour supprimer les réflexions parasites,
- dans un boîtier, un système d'éclairement et de formation d'image comprenant des surfaces inclinées et/ou décentrées, réfractantes et/ou réfléchissantes, ou des dispositifs constitués de surfaces optiques non inclinées ou non décentrées ayant un montage de type point noir dans le système d'éclairement,
dans lequel,
- sur un chemin de faisceaux de formation d'image, il est prévu au moins un miroir de formation d'image (11 ; 12) comprenant une optique de formation d'image, dans lequel, pour compenser les aberrations de l'optique de formation d'image, il est prévu au moins un élément optique pouvant être structuré ou adaptatif de type réfléchissant, réfractant ou électronique, par exemple un miroir (12) comportant des éléments réglables et adaptatifs (12a),
- et des moyens destinés à générer des signaux de commande sont prévus pour commander les éléments adaptatifs (12a) de l'au moins un élément optique (12), **caractérisé en ce que**
- il est prévu une ou plusieurs diodes électroluminescentes (LED) fonctionnant en mode continu et/ou impulsionnel en tant que source d'éclairement,
- et **en ce qu'**il est prévu des moyens destinés à générer des impulsions de lumière séquentielles dans divers domaines spectraux et destinés à effectuer une acquisition synchronisée d'images au moyen d'un capteur d'image et à effectuer un traitement d'image afin de représenter et de mémoriser une image en couleur ou d'autres combinaisons quelconques ainsi que des images individuelles monochromatiques.

2. Dispositif selon la revendication 1, **caractérisé en ce qu'**il est en outre prévu des moyens destinés à compenser les aberrations de l'oeil d'un patient (2).

3. Dispositif selon la revendication 1 ou 2, **caractérisé en ce que** l'au moins un élément du système optique est réalisé sous la forme d'une surface de forme libre.

4. Dispositif selon l'une quelconque des revendications 1 à 3, **caractérisé en ce qu'**il est prévu en tant que moyens destinés à générer des signaux de commande pour commander les éléments adaptatifs (12a) du miroir (12), au moins un capteur de front d'onde destiné à déterminer le front d'onde à l'intérieur du montage optique.

5. Dispositif selon la revendication 4, **caractérisé en ce que** l'au moins un capteur de front d'onde est prévu pour déterminer le front d'onde en temps réel.

6. Dispositif selon la revendication 4, **caractérisé en ce qu'**il est prévu sur un capteur d'image des moyens destinés à effectuer un traitement d'image au moyen d'un logiciel, à l'aide desquels des signaux de commande destinés à commander les éléments adaptatifs (12a) d'au moins un miroir (12) sont acquis.

7. Dispositif selon la revendication 1, **caractérisé en ce que** des moyens sont présents pour compenser sélectivement des aberrations chromatiques au moyen d'au moins un élément optique réglable qui modifie sa position en synchronisme avec les impulsions de lumière (par exemple une lentille à décalage pas à pas).

8. Dispositif selon l'une quelconque des revendications 1 à 7, **caractérisé en ce qu'**il est prévu des moyens destinés à compenser sélectivement des aberrations chromatiques au moyen d'éléments réglés en synchronisme avec les impulsions de lumière d'au moins un composant optique adaptatif.

9. Dispositif selon l'une quelconque des revendications 1 à 8, **caractérisé en ce qu'**au moins un élément optique est réalisé sous la forme d'une séparatrice de couleurs spectrale.

10. Dispositif selon l'une quelconque des revendications précédentes, **caractérisé en ce que** le chemin du faisceau de formation d'image et/ou du faisceau d'éclairement est incliné.

11. Utilisation d'un dispositif selon l'une quelconque des revendications 1 à 10 dans des appareils destinés à la coagulation par laser de la rétine à différentes longueurs d'onde, notamment pour la coagulation par laser multicolore.

12. Utilisation d'un dispositif selon l'une quelconque des revendications 1 à 10 dans des appareils destinés à effectuer une tomographie de cohérence optique (OCT, Optical Coherence Tomography),
et/ou dans des appareils destinés à mettre en oeuvre un diagnostic et une thérapie oculaire au moyen de lasers à impulsions ultracourtes, notamment au moyen d'impulsions de l'ordre de la femtoseconde,
et/ou dans des appareils d'imagerie hyperspectrale, par exemple d'imagerie multispectrale destinés au diagnostic de fonctions de la rétine,
et/ou dans des appareils de réflectométrie du fond de l'oeil,
et/ou dans des scanners ponctuels ou linéaires destinés au diagnostic et/ou à la thérapie de la rétine,
et/ou dans des appareils qui fonctionnent sur une base confocale, notamment dans des scanners à lasers confocaux.
